# FASCICULE DE BREVET EUROPEEN

(11) **EP 2 441 491 B1**
(45) Date de publication et mention de la délivrance du brevet: **09.01.2013**
(21) Numéro de dépôt: 11178010.2
(22) Date de dépôt: 18.08.2011
(51) Int. Cl.: A61N 1/372

(54) **Implant médical actif autonome, avec un circuit de réveil de l'alimentation sur réception d'impulsions transmises via les tissus interstitiels du corps**
Autonomes aktives medizinisches Implantat mit einem Aufwachstromkreis zur Stromzuführung beim Empfang von Impulsen, die vom interstitiellen Gewebe des Körpers übertragen werden
Standalone active medical implant, with a circuit for awakening the input on receiving pulses transmitted via the interstitial tissue of the body

(30) Priorité: 18.10.2010 FR 1058476
(43) Date de publication de la demande: 18.04.2012
(73) Titulaire: Sorin CRM SAS, 92143 Clamart Cedex (FR)
(72) Inventeur: Makdissi, Alaa, 75011 Paris (FR); Amara, Karima, 92330 Sceaux (FR); Ghildiyal, Ashutosh, 92140 Clamart (FR)
(74) Mandataire: Dupuis-Latour, Dominique

(56) Documents cités:
- US-A- 6 141 588
- US-A1- 2002 099 423
- US-A1- 2008 048 836
- US-A1- 2008 071 328

## Description

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les implants cochléaires, etc., ainsi que les dispositifs de mesure de pH ou encore d'impédance intracorporelle (telle que mesure d'impédance transpulmonaire ou d'impédance intracardiaque).

L'invention concerne plus particulièrement ceux de ces dispositifs qui mettent en oeuvre des capsules autonomes implantées et dépourvues de toute liaison physique à un dispositif principal implanté (tel qu'un boîtier de générateur d'impulsions de stimulation) ou non implanté (périphérique externe tel que programmateur ou dispositif de *monitoring* pour le suivi à distance du patient). La communication, conduite par les tissus interstitiels du corps, est alors du type dit "HBC" (*Human Body Communication*, communication par voie intracorporelle).

Ces capsules autonomes sont dénommées pour cette raison "capsules *leadless*", pour les distinguer des électrodes ou des capteurs disposés à l'extrémité distale d'une sonde (*lead*), cette sonde étant parcourue sur toute sa longueur par un ou plusieurs conducteurs reliant par voie galvanique l'électrode ou le capteur à un générateur connecté à l'extrémité opposée, proximale, de la sonde.

De telles capsules *leadless* sont par exemple décrites dans les US 2007/0088397 A1 et WO 2007/047681 A2 (Nanostim, Inc.) ou encore dans le US 2006/0136004 A1 (EBR Systems, Inc.).

Ces capsules *leadless* peuvent être notamment des capsules épicardiques, fixées à la paroi extérieure du coeur, ou bien des capsules endocavitaires, fixées à la paroi intérieure d'une cavité ventriculaire ou auriculaire. Leur fixation à la paroi cardiaque se fait habituellement au moyen d'une vis d'ancrage hélicoïdale saillante, prolongeant axialement le corps de la capsule et destinée à pénétrer dans le tissu cardiaque par vissage au site d'implantation.

Une telle capsule comprend des circuits de détection/stimulation pour recueillir des potentiels de dépolarisation du myocarde et/ou pour appliquer des impulsions de stimulation au site où est implantée la capsule. La capsule porte alors une électrode appropriée, qui peut être notamment constituée par une partie active de la vis d'ancrage. Elle peut également incorporer un ou plusieurs capteurs permettant de mesurer localement la valeur d'un paramètre tel que le niveau d'oxygène dans le sang, la pression cardiaque endocavitaire, l'accélération de la paroi cardiaque, l'accélération du patient come indicateur de l'activité etc.

L'invention n'est toutefois pas limitée à un type particulier de capsule, et elle est applicable indifféremment à tout type de capsule *leadless*, quelle que soit sa destination fonctionnelle.

Bien entendu, pour permettre l'échange de données à distance, les capsules *leadless* incorporent des moyens émetteurs/récepteurs de communication sans fil.

Plusieurs techniques ont été proposées pour assurer la communication sans fil entre ces capsules autonomes et un dispositif distant destiné à centraliser les informations recueillies par les capsules et à leur envoyer si nécessaire des commandes appropriées (ce dispositif peut être notamment un stimulateur, resynchroniseur ou défibrillateur implanté, un défibrillateur sous-cutané, ou un enregistreur longue durée).

Ainsi, le US 2006/0136004 A1 propose de transmettre les données par des ondes acoustiques se propageant à l'intérieur du corps. Cette technique est efficace et sans danger ; elle présente toutefois l'inconvénient de nécessiter une puissance d'émission relativement élevée compte tenu de l'atténuation des ondes acoustiques dans le corps, et ne permet qu'un débit de données relativement faible.

Le US 5 411 535 A propose une autre technique, basée sur l'utilisation d'ondes radiofréquence (RF). Ici encore, une puissance d'émission relativement importante est nécessaire, et l'atténuation de ces ondes par les tissus intracorporels est un obstacle important à leur propagation.

Une autre technique encore a été proposée par le US 4 987 897 A, mais il s'agit d'un échange de données avec un dispositif externe (programmateur), par voie transcutanée et non plus intracorporelle. Cette transmission est assurée à courte distance entre, d'une part, le boîtier d'un stimulateur implanté dans une poche sous-cutanée et, d'autre part, un programmateur externe posé contre la peau, à proximité du générateur. Les courants circulent donc au travers de la peau dans une région très éloignée des endroits sensibles, notamment à distance du myocarde, ce qui évite tout risque de perturbation des ondes de dépolarisation naturelle ou stimulée de ce dernier.

Le US 2007/0088397 A1 propose par ailleurs d'utiliser les impulsions de stimulation produites par une capsule comme véhicule pour la transmission de données préalablement recueillies ou élaborées par la capsule. Pour cela, l'impulsion, au lieu de présenter une variation monotone de tension, est interrompue de manière contrôlée pendant des durées très brèves de manière à créer dans le profil de l'impulsion de très étroits créneaux dont la succession correspond à un codage binaire de l'information à transmettre.

Quelle que soit la technique mise en oeuvre, le traitement d'un signal HBC recueilli côté capsule réceptrice nécessite une énergie non négligeable par rapport aux ressources énergétiques dont dispose cette capsule. Compte tenu de son caractère autonome, la capsule ne peut en effet faire appel qu'à ses ressources propres telles qu'un circuit de récupération d'énergie (par le mouvement de la capsule) et/ou une petite batterie intégrée.

La gestion de l'énergie disponible est donc un point crucial pour le développement des techniques de communication HBC entre capsules autonomes.

Or la communication n'est pas permanente, de sorte que les circuits actifs de traitement (amplification, numérisation, décodage, analyse des signaux, ...) restent inutilement alimentés, avec une incidence négative sur l'autonomie de la capsule, en l'absence de mise en sommeil spécifique. Mais d'autre part, s'il est prévu une mise en sommeil des circuits de réception, il devient impossible de détecter l'arrivée d'un signal HBC.

Il existe de ce fait le besoin de disposer d'un système permettant de réveiller en cas de besoin les capsules, avec un temps de latence relativement faible pour ne pas obérer le débit de la communication HBC, ni la réactivité de la capsule aux commandes qui lui sont adressées par ce biais.

Une technique qui a été envisagée consiste à laisser en sommeil les circuits récepteurs et à les réveiller à intervalles réguliers pour détecter la présence éventuelle de signaux de communication HBC envoyés d'une autre capsule ou d'un autre dispositif. Cette technique oblige à trouver un compromis entre une périodicité faible des réveils - qui économise l'énergie mais diminue la réactivité - et inversement une périodicité élevée - qui améliore la réactivité mais ne réduit que faiblement la consommation moyenne de la capsule sur le long terme.

Le US 2008/071328 A1 propose un dispositif *leadless* implanté pourvu d'un circuit de réveil activé par des signaux RF ou acoustiques (donc des signaux non-HBC) émis depuis un autre dispositif distant, ces signaux pouvant être codés en fonction par exemple d'un schéma d'adressage particulier permettant de ne réveiller sélectivement qu'un implant particulier choisi. L'utilisation de signaux non-HBC pour le réveil requiert toutefois la mise en oeuvre de circuits de communication spécifiques aussi bien côté récepteur que côté émetteur.

L'approche de l'invention est différente. Elle repose essentiellement sur l'utilisation d'un circuit de réveil comportant un récepteur passif (c'est-à-dire sans amplificateur ni autre circuit avec une consommation notable par rapport au bilan énergétique global de la capsule), en permanence capable de détecter l'arrivée d'un signal HBC. Ce n'est que lorsqu'un tel signal aura été détecté que le circuit réveillera le système et activera les amplificateurs et autres circuits actifs consommateurs d'énergie.

L'une des difficultés majeures de cette approche tient au faible niveau des signaux à détecter par le circuit de réveil, compte tenu de l'atténuation rapide des impulsions électriques se propageant au sein des tissus interstitiels du corps.

De plus, les impulsions de communication HBC peuvent se trouver mêlées à des signaux électriques parasites de niveau relativement élevé, tels que les myopotentiels naturels du corps et les ondes de dépolarisation cardiaque se propageant au sein du myocarde - d'où un rapport signal/bruit dégradé. Il y a également lieu de discriminer pour les exclure les impulsions de stimulation délivrées par un générateur implanté, appliquées localement en certains sites du myocarde mais qui se diffusent ensuite autour du site de stimulation dans une large zone avant d'être substantiellement atténuées.

La technique mise en oeuvre doit donc être capable de prendre en compte ces aspects et assurer un filtrage et une discrimination efficace de tous les signaux parasites.

En ce qui concerne plus particulièrement l'atténuation des signaux par les tissus du corps, dans la bande de fréquences 500 kHz-10 MHz (bande B) qui est celle qui présente une atténuation minimale par les tissus interstitiels du corps, l'atténuation dans cette bande de fréquences varie typiquement entre 10 dB et 40 dB, selon la distance entre émetteur et récepteur, l'écartement entre les électrodes respectives du couple d'électrodes de l'émetteur ou du récepteur, et la surface de ces électrodes. Une valeur d'atténuation typique est de 20 dB à 1 MHz pour une distance de 10 à 12 cm entre émetteur et récepteur.

Mais dans certaines conditions extrêmes, cette atténuation peut atteindre 60 dB, de sorte que le signal de réveil envoyé à la capsule se trouvera noyé dans un bruit de fond important, donc avec à la fois une très faible amplitude de l'impulsion à détecter et un rapport signal/bruit très médiocre.

Le but de l'invention est de proposer une technique permettant de remédier à ces divers inconvénients, qui notamment :
- préserve la relativement faible autonomie des capsules *leadless*, dépendantes d'un système d'auto-alimentation intégré ;
- ne risque pas d'être perturbée par les signaux électriques parasites présents au sein des tissus du corps, notamment les perturbations par les myopotentiels et potentiels cardiaques présents dans l'organisme, ni par les impulsions de stimulation éventuellement délivrées par un implant ;
- ne nécessite pas de circuits complexes pour sa mise en oeuvre ;
- et présente une latence minimale, de manière à assurer une réactivité élevée de la capsule *leadless* par rapport à l'instant de l'envoi de l'impulsion de réveil.

L'invention propose à cet effet un ensemble du type général divulgué par le US 2008/071328 A1 précité, c'est-à-dire comprenant au moins deux dispositifs médicaux implantables actifs, ces dispositifs comportant des moyens pour communiquer entre eux sans fil. L'ensemble comprend au moins un dispositif émetteur et au moins un dispositif récepteur. Le dispositif émetteur comprend des moyens émetteurs aptes à générer des trains d'impulsions modulées formés d'une succession d'impulsions et à appliquer ces impulsions à des électrodes du dispositif émetteur, ces moyens émetteurs étant en outre aptes, préalablement à l'initiation d'une communication avec le dispositif récepteur, à générer un signal spécifique de réveil, configuré selon un motif caractéristique prédéterminé. Le dispositif récepteur comprend des moyens récepteurs aptes à filtrer, amplifier et démoduler des trains d'impulsions recueillis aux bornes d'électrodes du dispositif récepteur, ces moyens récepteurs étant des moyens sélectivement activables d'un état de sommeil, où ils ne sont pas alimentés par une source d'énergie du dispositif récepteur, vers un état opérationnel, où ils sont alimentés et aptes à filtrer, amplifier et démoduler les impulsions recueillies. Le dispositif récepteur comprend également, outre les moyens récepteurs, des moyens de réveil) aptes à discriminer la réception d'un signal spécifique de réveil configuré selon ledit motif caractéristique prédéterminé et, en réponse, faire basculer les moyens récepteurs de l'état de sommeil vers l'état opérationnel.

De façon caractéristique de l'invention : ladite communication sans fil est une communication par voie intracorporelle au moyen de signaux constitués desdites impulsions électriques, aptes à être conduites par les tissus interstitiels du corps ; ledit signal spécifique de réveil généré par lesdits moyens émetteurs est un train desdites impulsions configuré selon un motif d'impulsions caractéristique prédéterminé ; et les moyens de réveil comprennent des moyens détecteurs et un comparateur à hystérésis. Les moyens de réveil sont très avantageusement dépourvus de circuit amplificateur. La consommation permanente des moyens de réveil est typiquement inférieure à 10 nW.

Par ailleurs, les impulsions du train d'impulsions spécifique de réveil sont de préférence des impulsions biphasiques comprenant chacune une alternance positive et une alternance négative.

Dans un premier mode de réalisation de l'invention, les moyens émetteurs génèrent le motif caractéristique sous forme d'une impulsion initiale d'amplitude élevée pour le réveil, suivie d'une succession d'impulsions de moindre amplitude pour la communication avec le dispositif récepteur. Ils peuvent également prévoir un délai de garde séparant l'impulsion initiale d'amplitude élevée de la succession d'impulsions de moindre amplitude. Les moyens de réveil comprennent avantageusement un filtre passe-haut amont d'atténuation des signaux physiologiques parasites présents dans les tissus interstitiels du corps, un détecteur de niveau de crête, ou crête-à-crête, des impulsions reçues, et des moyens pour inhiber le basculement des moyens récepteurs de l'état de sommeil vers l'état opérationnel sur détection d'une impulsion reçue de largeur supérieure à une limite supérieure prédéterminée.

Dans un second mode de réalisation de l'invention, les moyens émetteurs génèrent le motif caractéristique sous forme d'une séquence spécifique d'impulsions d'amplitude uniforme codant une valeur binaire prédéterminée formant une code de réveil. Les moyens de réveil comprennent des moyens pour individualiser les impulsions reçues, décoder la valeur binaire codée par ces impulsions reçues, et vérifier si cette valeur décodée concorde ou non avec une valeur prédéterminée mémorisée dans le dispositif récepteur.

De préférence, les moyens émetteurs génèrent la séquence spécifique d'impulsions sous formes d'une pluralité de salves distinctes d'impulsions, certaines d'entre ces salves étant constituées d'un premier nombre d'impulsions codant un '1' binaire et les autres salves étant constituées d'un second nombre d'impulsions, différent du premier nombre, par exemple double, codant un '0' binaire. Les moyens de réveil comprennent un détecteur d'enveloppe délivrant en sortie une série de valeurs binaires obtenues chacune par intégration d'une salve d'impulsions correspondante reçue, et des moyens numériques de comparaison de cette série de bits à ladite valeur prédéterminée mémorisée. Le détecteur d'enveloppe peut avantageusement comprendre un circuit multiplicateur de tension.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 illustre de façon schématique un ensemble de dispositifs médicaux comprenant notamment des capsules *leadless*, implantées au sein du corps d'un patient.
La Figure 2 montre plus précisément la manière d'implanter ces capsules *leadless* sur la paroi interne ou externe du myocarde.
La Figure 3 est un schéma par blocs fonctionnels montrant les différents étages constitutifs d'une capsule *leadless*.
La Figure 4 illustre schématiquement les éléments côté émetteur et côté récepteur nécessaire à la mise en oeuvre de l'invention.
Les Figures 5a et 5b sont des chronogrammes montrant l'allure d'une impulsion de réveil selon un premier mode de réalisation de l'invention, respectivement telle qu'émise et telle que reçue.
La Figure 6 illustre schématiquement les différents étages d'un circuit de réveil selon ce mode de réalisation de l'invention, circuit apte à détecter et analyser une impulsion telle que celle de la Figure 5b.
La Figure 7 montre l'allure de deux impulsions de réveil émises selon un second mode de réalisation de l'invention.
La Figure 8 illustre schématiquement les étages d'un circuit de réveil selon ce second mode de réalisation de l'invention, circuit apte à détecter et analyser les impulsions de la Figure 7 reçues côté récepteur.
La Figure 9 présente, sur un même chronogramme, le signal de réveil brut, tel que reçu, et le signal démodulé correspondant obtenu en sortie du détecteur d'enveloppe du circuit de la Figure 8.

On va maintenant décrire un exemple de réalisation de l'invention.

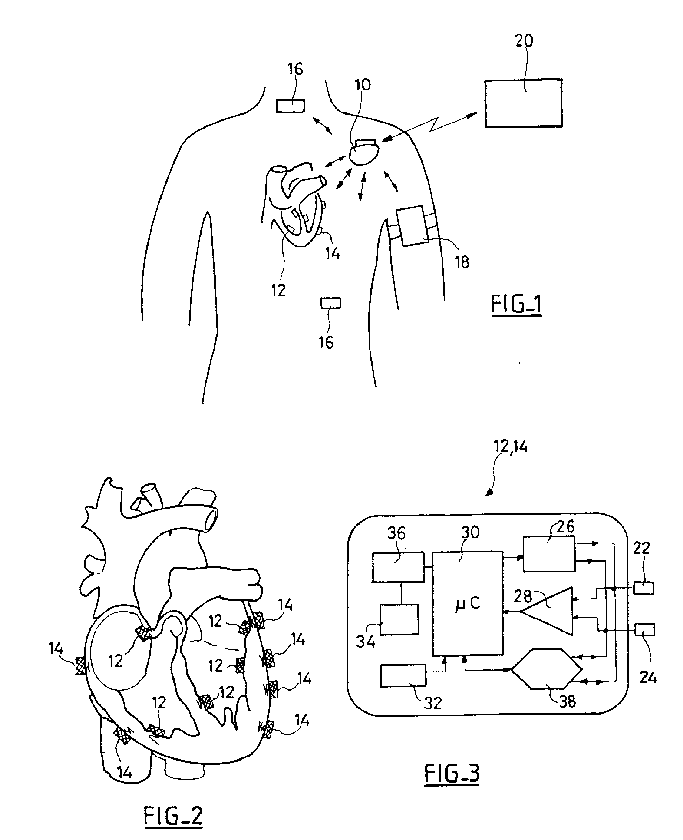

Sur la Figure 1 on a illustré un ensemble de dispositifs médicaux implantés au sein du corps d'un patient, communiquant entre eux sans fil par voie "HBC" (*Human Body Communication*, communication par voie intracorporelle).

Le patient est équipé par exemple d'un implant 10 tel qu'un défibrillateur/stimulateur/resynchroniseur implanté, ou un défibrillateur de type sous-cutané, ou encore un enregistreur longue durée. Ce dispositif implanté 10 est le dispositif maître d'un réseau comportant une pluralité de dispositifs esclaves 12 à 18 avec lesquels il est susceptible d'entrer en communication par voie HBC. Ces dispositifs peuvent notamment inclure des capsules intracardiaques 12 ou épicardiques 14 implantées directement sur le coeur du patient, d'autres dispositifs 16 tels que capteurs de myopotentiels ou dispositifs de stimulation neurologique, et éventuellement un dispositif externe 18 disposé sur un brassard et pourvu d'électrodes en contact avec la peau. Le dispositif 10 peut également être utilisé en tant que passerelle avec le monde extérieur pour communiquer avec un périphérique externe 20 du type programmateur ou dispositif de télétransmission de données avec lequel il pourra communiquer notamment par télémétrie RF dans la bande MICS (*Medical Implants Communication System*) 402-405 MHz, ou les bandes banalisées publiques ISM (Industriel, Scientifique et Médical) 863-870 MHz, 902-928 MHz et 2,4 GHz utilisées par les dispositifs médicaux.

Chacun des dispositifs 10 à 18 est muni d'au moins un couple d'électrodes qui se trouvent en contact direct avec les tissus du corps pour les dispositifs implantés, ou en contact avec la peau pour le dispositif externe 18.

Sur la Figure 2, on a représenté un exemple de capsules de type *leadless* implantées soit sur la partie antérieure du myocarde, à l'intérieur d'une cavité auriculaire ou ventriculaire (capsules endocavitaires 12), soit sur une paroi externe de ce même myocarde (capsules épicardiques 14). Ces capsules, qui sont par exemple décrites dans les US 2007/0088397 A1, WO 2007/047681 A2 et US 2006/0136004 A1 précités, sont fixées à la paroi cardiaque au moyen d'une vis d'ancrage saillante destinée à pénétrer dans le tissu cardiaque par vissage au site d'implantation. La vis peut être soit une vis passive, ne servant qu'à la fixation de la capsule, soit une vis active, servant à recueillir les signaux de dépolarisation se propageant dans les tissus du myocarde et/ou à délivrer des impulsions de stimulation au site d'implantation, de façon localisée.

La Figure 3 illustre de façon schématique les différents circuits internes des capsules 12, 14 (et, *mutatis mutandis*, des autres éléments implantés prévus pour communiquer entre eux par la technique de l'invention). Chaque capsule comporte un couple d'électrodes 22, 24, l'une d'entre elles pouvant d'ailleurs être constituée par la vis d'ancrage dans le tissu du coeur. Ces électrodes sont reliées à un circuit 26 générateur d'impulsions de stimulation (pour une capsule active incorporant cette fonction) et/ou à un circuit de détection 28 servant au recueil des potentiels de dépolarisation recueillis entre les électrodes 22 et 24.

Un circuit central 30 inclut l'ensemble de l'électronique permettant de piloter les diverses fonctions de la capsule, la mémorisation des signaux recueillis, etc. Il comprend un microcontrôleur et un oscillateur générant les signaux d'horloge nécessaires au fonctionnement du microcontrôleur et à la communication. Il peut également contenir un convertisseur analogique/numérique et une mémoire de stockage numérique.

La capsule peut également être pourvue d'un capteur 32 tel qu'un capteur d'accélération, de pression, un capteur hémodynamique, de température, de saturation en oxygène, etc. La capsule est alimentée par une petite batterie ou un circuit de récupération d'énergie 34 alimentant l'ensemble des circuits via un étage de gestion d'énergie 36.

Les électrodes 22 et 24 sont également reliées à un circuit modulateur/démodulateur 38 couplé au circuit processeur central 30 et apte à émettre et/ou recevoir des impulsions servant à la communication sans fil HBC. Ainsi, selon que les circuits de stimulation (module 26) et de recueil (module 28) sont présents ou non, les électrodes 22, 24 peuvent assurer une simple, double ou triple fonction, à savoir : stimulation et/ou recueil des potentiels cardiaques (le cas échéant) ; et/ou transmission des informations suivies par le capteur 32 (le cas échéant) ; et émission/réception pour la communication HBC (en tout état de cause).

L'invention concerne plus particulièrement l'étage de gestion d'énergie 36, et notamment la manière dont celui-ci peut être utilisé pour alimenter sélectivement les circuits actifs consommateurs d'énergie (amplificateur 28, microcontrôleur 30, circuit d'émission/réception HBC 38, etc.), seulement lorsqu'ils seront nécessaires. Le reste du temps ces circuits seront mis en sommeil donc ne consommeront aucune énergie.

Cet étage de gestion d'énergie comprend pour cela un circuit de réveil seulement constitué de composants passifs ou à très faible consommation (assimilables à des composants passifs du point de vue du bilan énergétique), comme on le décrira en détail par la suite,.

Ce circuit de réveil a pour fonction de détecter, pendant la période de sommeil des autres circuits (les circuits actifs), des trains d'impulsions spécifiques de réveil, configurés selon un motif d'impulsions caractéristique prédéterminé.

Ces trains d'impulsions constituent des commandes de réveil générées à distance par un dispositif, tel qu'un stimulateur *leadless* ou un appareil sous-cutané, chargés de surveiller l'activité cardiaque et de générer des impulsions de stimulation, de resynchronisation et/ou de défibrillation, ou encore par une autre capsule du système. Le dispositif distant peut par exemple jouer un rôle de concentrateur ou de dispositif maître dans une architecture de réseau sans fil en étoile, dont les diverses capsules *leadless* seront des dispositifs esclaves (pour une communication dans ce sens - pour une communication en sens inverse, les rôles seront inversés).

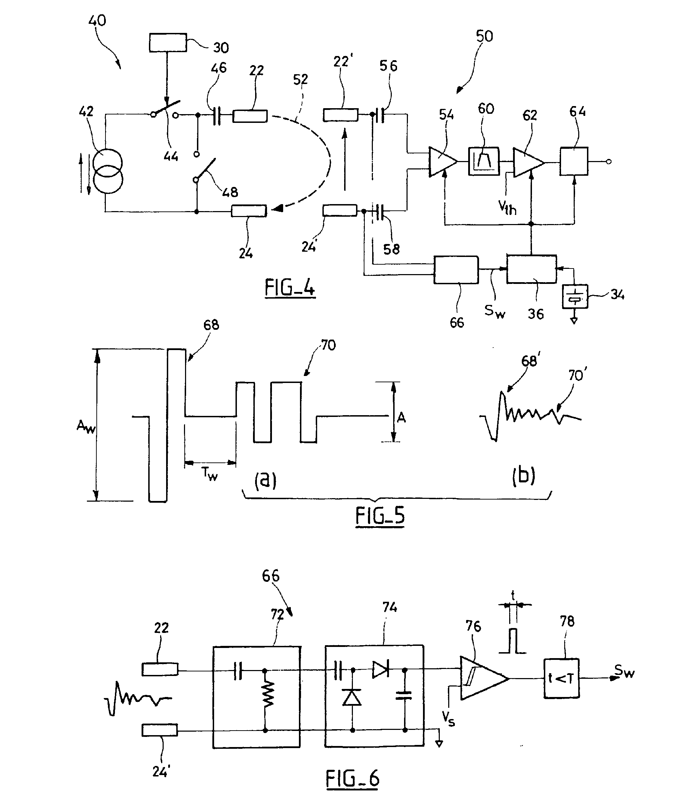

La Figure 4 illustre schématiquement les éléments utilisés par ce dispositif maître (ou "dispositif émetteur") et par la capsule à réveiller (ou "dispositif récepteur") pour la mise en oeuvre de l'invention.

Les circuits du dispositif émetteur 40 comprennent une source de courant (ou de tension) 42, ajustable sur commande pour générer une impulsion de tension de valeur appropriée. Le circuit central de commande 30 commande l'ouverture et la fermeture d'interrupteurs, en particulier la fermeture de l'interrupteur 44 afin d'injecter le courant dans un sens prédéterminé et sur un intervalle de temps prédéterminé. Le courant injecté 52 circulera (via le condensateur de liaison 46, partagé ou non avec l'étage de stimulation, permettant d'éviter tout envoi de tension continue sur les électrodes) au travers du corps du patient depuis l'une des électrodes 22 jusqu'à l'autre électrode 24. L'interrupteur 48 permet de décharger ensuite le condensateur 46 de la charge résiduelle due aux erreurs de compensation des impulsions positives et négatives. Après avoir injecté une première alternance, on procède de même pour injecter l'alternance suivante en inversant le sens du courant, pour obtenir les impulsions biphasiques requises, que l'on décrira plus bas.

Il est en effet souhaitable de générer des impulsions biphasiques, afin de réduire au maximum les charges résiduelles injectées dans le coeur, et réduire également la corrosion éventuelle des matériaux. Dans les exemples illustrés, ces impulsions sont délivrées sous forme de deux alternances successives, positive et négative (ou l'inverse), de formes carrées et symétriques (même amplitude en valeur absolue, même durée pour les deux alternances). D'autres formes d'onde sont cependant envisageables, et l'exemple exposé ici n'est pas limitatif.

Toujours sur la Figure 4, la référence 50 désigne les moyens récepteurs du dispositif récepteur : le courant 52 circulant dans le corps génère entre les électrodes 22' et 24' du récepteur une différence de potentiel qui est appliquée à un étage amplificateur 54 via les condensateurs de liaison 56 et 58 permettant d'éliminer toute composante continue. Le signal amplifié résultant est appliqué à un filtre passe-bande 60 afin de filtrer les signaux parasites hors de la bande pertinente, et le signal filtré obtenu est appliqué à un comparateur à seuil 62 et à un étage démodulateur 64.

De façon caractéristique de l'invention, les circuits 54, 62 et 64, ainsi que les autres circuits actifs (consommateurs d'énergie) du dispositif récepteur ne seront alimentés que lorsque cela sera nécessaire, afin de réduire la consommation énergétique moyenne du dispositif.

Pour cela, l'invention prévoit de relier aux électrodes 22' et 24' un circuit de réveil 66 apte à détecter et à discriminer l'arrivée d'impulsions spécifiques de réveil sur ces électrodes et à délivrer dans ce cas un signal S_{w} de *wake-up* ou commande de réveil appliqué en entrée à l'étage de gestion d'énergie 36 pour mettre sous la tension de la source d'énergie 34 les circuits consommateurs 54, 62, 64, ... auparavant en sommeil.

On va maintenant décrire deux modes de réalisation possibles de l'invention, spécifiquement adaptés à des qualités différentes du canal de communication entre dispositif émetteur et dispositif récepteur, qualités tenant essentiellement : (i) à l'atténuation du signal entre émetteur et récepteur, et (ii) au rapport signal/bruit plus ou moins dégradé par des signaux parasites présents dans les tissus interstitiels.

Le premier mode de réalisation est illustré sur les Figures 5 et 6.

Ce mode de réalisation est optimisé pour des canaux de communication présentant une faible atténuation et un bon rapport signal/bruit (par "faible atténuation" on entendra une atténuation comprise entre x10 et x100, soit 20 à 40 dB).

Dans ce cas de figure, on utilisera pour déclencher le réveil un motif d'impulsions caractéristique tel que celui illustré Figure 5a, comprenant une impulsion 68 (biphasique) de forte amplitude A_{w}, formant commande de réveil, suivie d'une succession d'impulsions 70 (biphasiques également) de moindre amplitude A, pour la communication HBC avec le dispositif récepteur.

On pourra par exemple générer du côté dispositif émetteur une impulsion biphasique de ± 1,5 V (soit une amplitude crête-à-crête A_{w} = 3 V) ce qui donnera, pour une atténuation comprise entre 20 et 40 dB, une impulsion d'amplitude comprise entre ± 150 mV et ± 15 mV environ du côté du dispositif récepteur.

Le signal reçu sera comparable à celui illustré Figure 5b, qui est une copie atténuée et filtrée (par les tissus du corps) du signal originel émis, avec une première impulsion 68' d'amplitude supérieure et une série d'impulsions 70' qui lui font suite.

La Figure 6 illustre les différents étages du circuit de réveil 66 : celui-ci comprend un filtre passe-haut amont 72 recevant le signal capté sur les électrodes 22' et 24' et délivrant en sortie le signal filtré à un étage détecteur 74, qui peut être un simple détecteur d'amplitude ou, de préférence, un détecteur crête-à-crête, comme celui illustré sur la figure (l'utilisation d'un détecteur crête-à-crête permet d'améliorer la sensibilité du système de détection). La sortie du détecteur 66 est appliquée à l'entrée d'un comparateur à hystérésis 76 dont le basculement permettra de générer le signal S_{w} destiné à réveiller les circuits actifs du dispositif récepteur.

Le filtre passe-haut 72 permet de filtrer les signaux physiologiques pour ne pas les rendre visibles par les circuits détecteurs et comparateurs disposés en amont, et éviter ainsi de faux réveils. Ce filtre 72 peut être un circuit résonant passif accordé à la fréquence des impulsions qui amplifie le signal entre les électrodes, ou un simple filtre passe-haut du premier ordre réalisé par un circuit RC, les valeurs de R et de C étant choisies de manière à atténuer largement les signaux physiologiques par rapport à l'hystérésis du comparateur 76. Ces signaux physiologiques parasites sont essentiellement constitués des myopotentiels du corps et des ondes de dépolarisation (signaux EGM) qui se propagent dans le myocarde, qu'elles soient d'origine spontanée ou stimulée. À titre d'exemple, un filtre passe-haut passif du premier ordre avec une fréquence de coupure de 1 MHz atténue de 80 dB les signaux EGM dont le contenu spectral est dans la bande 0-100 Hz, de sorte qu'un signal EGM de 40 mV ne contribuera pas pour plus de 4 µV au signal reçu sur les électrodes 22' et 24' (signal dont on a vu qu'il avait généralement une valeur de plusieurs dizaines de millivolts).

En ce qui concerne le détecteur d'amplitude 74, celui-ci est avantageusement un détecteur crête-à-crête, réalisé sous forme d'un redresseur double composé de deux condensateurs et deux diodes. La tension à la sortie du détecteur 74 sera égale à Vₘₐₓ - Vₘᵢₙ - 2 V_{d}, où Vₘₐₓ est le maximum du signal reçu, Vₘᵢₙ le minimum du signal reçu après filtrage, et V_{d} la tension de seuil des diodes. Si cette tension de seuil est faible, on pourra réaliser un détecteur crête-à-crête sans pertes, bien connu de l'état de la technique sous le nom de "diode active".

En ce qui concerne le comparateur 76, son seuil Vₛ est de préférence défini à Vₛ = 0 V (la comparaison par rapport à un niveau 0 V simplifie l'électronique associée au circuit, par rapport à un comparateur à une tension prédéfinie). La valeur de l'hystérésis dépendra du niveau de bruit de réception. Dans le cas de l'exemple indiqué plus haut (impulsion de réveil générée à une tension de ± 1,5 V, avec 40 dB d'atténuation), pour une tension crête-à-crête de 30 mV disponible en sortie du détecteur 74, on pourra par exemple paramétrer l'hystérésis du comparateur 76 sur une valeur de l'ordre de 15 mV.

Un autre signal parasite qu'il convient d'éliminer est celui constitué par les stimulations électriques appliquées au coeur, qui présentent une amplitude élevée risquant de déclencher à tort le circuit de réveil. Pour empêcher ce déclenchement intempestif, on peut ajouter un critère supplémentaire pour valider le réveil et générer le S_{w}.

Cette condition consiste à vérifier (étage 78) que la largeur *t* du signal logique généré en sortie du comparateur 76 est inférieure à une durée prédéterminée T, proche de la durée de l'impulsion de réveil (qui, concrètement, est beaucoup plus courte qu'une impulsion de stimulation).

Pour tester cette condition, une première solution consiste à compter la durée entre le front descendant et le front montant de l'impulsion 68' reçue, au moyen d'un compteur (horloge) déjà disponible dans le dispositif récepteur, à condition que le cycle de ce compteur soit plus court que la durée de l'impulsion de réveil. Au bout d'un certain temps limite (*time-out*), si le front descendant ne survient pas, la détection sera invalidée et le signal S_{w} ne sera pas généré.

Une autre technique de validation du signal de réveil consiste à intégrer le signal en sortie du comparateur 76, par exemple au moyen d'un simple circuit RC, et à comparer le résultat à un seuil prédéterminé.

Dans l'un ou l'autre cas, il faudra attendre un certain temps (celui du *time-out*) pour pouvoir discriminer un signal de réveil (impulsion brève) d'une impulsion de stimulation (impulsion longue). En pratique, une durée de l'ordre de trois à quatre fois la durée de l'impulsion de réveil 68 constitue un compromis acceptable, qui ne réduit pas trop la réactivité du circuit de réveil.

Le signal S_{w} produit par le circuit de réveil 66 provoque la mise sous tension des divers circuits actifs du dispositif récepteur, qui pourront alors recevoir et analyser les impulsions HBC. Pour tenir compte du temps de démarrage de ces circuits de réception à leur lise sous tension, il convient de prévoir un temps de latence T_{w} (Figure 5a) entre l'impulsion de réveil 68 et les impulsions de communication HBC 70.

Par ailleurs, si la qualité du canal est suffisamment bonne pour permettre la détection sans amplification des impulsions HBC de faible amplitude, on pourra utiliser les étages de filtrage 72, de détection 74 et de mise en forme (comparateur) 76 du circuit de réveil 66 également pour l'échange des données HBC. On réduira ainsi la complexité et la taille du dispositif récepteur, en utilisant un seul et même circuit pour le réveil du récepteur et le recueil des données HBC.

Le circuit de réveil que l'on vient de décrire nécessite peu de ressources énergétiques, car les étages 72 et 74 sont réalisés avec des circuits purement passifs. Le seul élément consommateur est le comparateur 76, mais les technologies CMOS actuelles permettent de réaliser un comparateur à hystérésis activé en permanence avec une consommation moyenne extrêmement minime, de l'ordre de quelques dizaines ou centaines de nanowatts (à titre de comparaison, un amplificateur de signal consomme environ 1000 fois plus). Cette quantité est négligeable par rapport au bilan énergétique global d'une capsule dans son état opérationnel avec tous ses circuits actifs, de l'ordre de 5 à 10 µW.

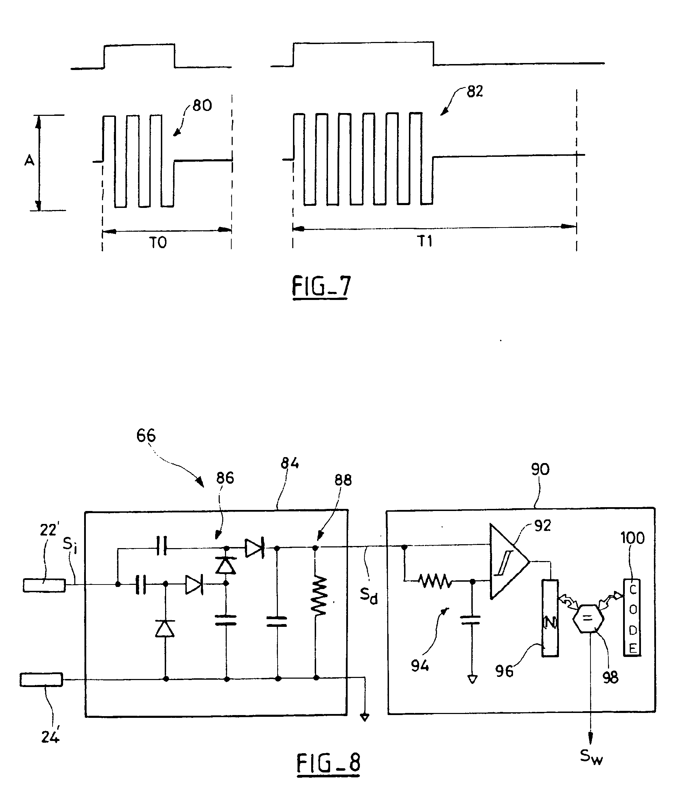

Les Figures 7 et 8 illustrent un deuxième mode de réalisation de l'invention, qui est notamment approprié aux situations dans lesquelles le premier mode de réalisation n'est pas applicable, tels que les cas de forte atténuation (jusqu'à x1000, soit 60 dB) et/ou de rapport signal/bruit fortement dégradé.

Le signal de réveil proposé est, comme illustré Figure 7, constitué à partir de salves d'impulsions telles que 80 ou 82, qui codent un bit de signal binaire, respectivement '0' ou '1'. Il s'agit d'une modulation d'amplitude de type ASK, en "tout ou rien" les impulsions étant des amplitudes biphasiques d'amplitude A (crête-à-crête) constante. Pour une impulsion de durée élémentaire Tₛ, un zéro binaire sera codé par une salve de n impulsions biphasiques, par exemple *n* = 3 impulsions comme illustré sur la Figure 7, suivies par un signal nul pendant une durée égale *n*.Tₛ. Les uns binaires sont codés par une salve 82 de 2*n* impulsions biphasiques (*n* = 6 impulsions dans l'exemple illustré), suivies par un signal nul pendant 2*n*.Tₛ.

Cette modulation est en fait une modulation double : en bande de base, la modulation est une modulation en largeur d'impulsions (PWM) dans laquelle la durée des zéros est égale à T₀ (*n* impulsions puis signal nul de durée *n*.Ts) et la durée des uns est égale à T₁ = 2T₀. On module ensuite l'amplitude du signal résultant (ligne du haut de la Figure 7) en bande de base par un motif en forme de salve d'impulsions, pour obtenir les deux salves 80 ou 82 (ligne du bas de la Figure 7).

La Figure 8 illustre un exemple de circuit de réveil 68 permettant de détecter et discriminer ces salves d'impulsions.

Ce circuit comporte tout d'abord un étage détecteur d'enveloppe 84, comprenant avantageusement un multiplicateur de tension 86, suivi d'un redresseur et d'un filtre passe-bas passif 88, par exemple un filtre passe-bas RC du premier ordre. Sur l'exemple illustré Figure 8, on a représenté un multiplicateur par quatre réalisé avec des condensateurs et des diodes à faible seuil, montage réalisable en technologie CMOS standard. Ce multiplicateur a pour effet de stocker aux bornes du condensateur de sortie (condensateur de l'étage 88) une tension égale à quatre fois l'amplitude du signal reçu sur les électrodes 22' et 24', réalisant ainsi une amplification de tension passive (le fait de disposer d'un signal alternatif - la salve d'impulsions - permet en effet de réaliser une telle amplification sans consommation d'énergie).

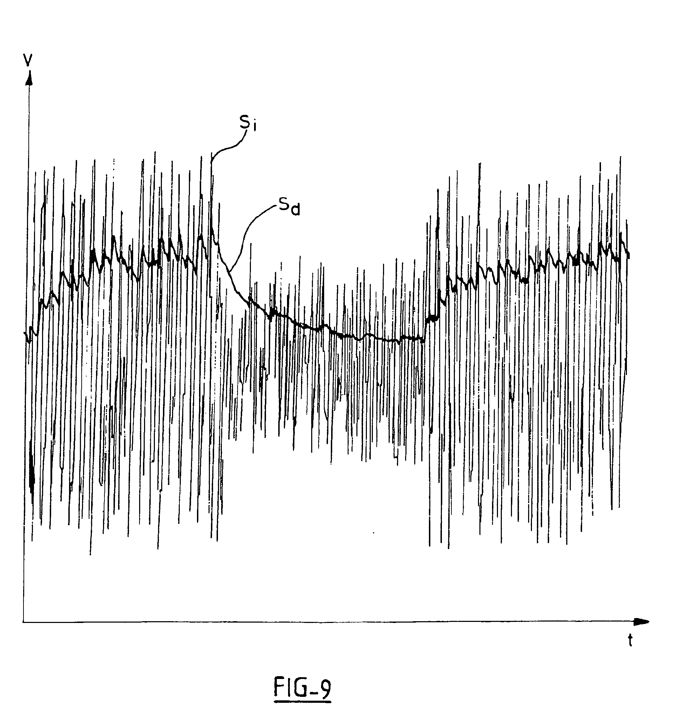

La Figure 9 montre le signal démodulé S_{d}, obtenu en sortie du détecteur d'enveloppe 84, par rapport au signal d'entrée Sᵢ, fortement bruité, recueilli sur les électrodes 22' et 24'.

Le signal Sᵢ issu du détecteur d'enveloppe 84 est appliqué à un décodeur 90 comprenant, comme dans le premier mode de réalisation, un comparateur à hystérésis 92 recevant sur l'une de ses entrées le signal S_{d} et dont la tension de référence appliquée à l'autre entrée est, avantageusement, un seuil dynamique constitué par la valeur moyenne du signal S_{d}, obtenue par un circuit intégrateur RC 94. Ceci permet d'éviter le paramétrage du décodeur 90 en fonction de la qualité du canal de transmission (atténuation et rapport signal/bruit).

En d'autres termes, au lieu d'une impulsion de forte amplitude comme dans le premier mode de réalisation, dans ce second mode de réalisation on envoie plusieurs impulsions d'amplitude uniforme, et on applique une intégration à la succession de ces impulsions pour aboutir à un résultat comparable.

Le décodage du signal détecté se fait sans horloge, de façon asynchrone, en stockant les bits successifs reçus et décodés dans un registre à décalage 96 de taille N prédéfinie. A la fin de la réception de la séquence de bits, le contenu de ce registre à décalage est comparé par un comparateur 98 à une valeur numérique codée de référence, conservée dans un registre 100 du dispositif. En cas de concordance, le signal S_{w} est généré pour réveiller les circuits actifs du dispositif, de la même manière que pour le premier mode de réalisation. Contrairement au premier mode de réalisation, il n'y a pas besoin dans ce cas de discriminer une impulsion de communication d'une impulsion de stimulation car il est impossible d'obtenir la valeur numérique de référence du registre 100 à partir d'une stimulation.

La valeur numérique stockée dans la mémoire 100 peut être un code de réveil générique (pour un réveil indifférencié de tous les implants du système), ou un code spécifique à chaque implant, de manière que le dispositif émetteur maître puisse réveiller sélectivement tel ou tel implant du système.

Dans ce second mode de réalisation, la qualité de détection du signal de réveil dépend de la durée d'un bit et non pas seulement du rapport signal/bruit. On peut ainsi augmenter le nombre d'impulsions codant un bit (*n* bits pour coder un '0" et 2*n* bits pour coder un '1') et augmenter en même temps la constante de temps (temps d'intégration) du détecteur d'enveloppe, pour augmenter la qualité de réception - avec en contrepartie une réduction du débit de communication.

Ici encore, les circuits sont tous des circuits passifs, à l'exception des comparateurs 92 et 98, dont la consommation est toutefois extrêmement minime, comme cela a été expliqué plus haut à propos du premier mode de réalisation.

## Revendications

1. Un ensemble comprenant au moins deux dispositifs médicaux implantables actifs, ces dispositifs (10, 12, 14, 16, 18) comportant des moyens pour communiquer entre eux sans fil,
cet ensemble comprenant au moins un dispositif émetteur et au moins un dispositif récepteur, où :
- le dispositif émetteur comprend des moyens émetteurs (40) aptes à générer des trains d'impulsions modulées formés d'une succession d'impulsions et à appliquer ces impulsions à des électrodes (22, 24) du dispositif émetteur,
lesdits moyens émetteurs (40) étant en outre aptes, préalablement à l'initiation d'une communication avec le dispositif récepteur, à générer un signal spécifique de réveil, configuré selon un motif caractéristique prédéterminé ; et
- le dispositif récepteur comprend des moyens récepteurs (50) aptes à filtrer, amplifier et démoduler des trains d'impulsions recueillis aux bornes d'électrodes (22', 24') du dispositif récepteur,
lesdits moyens récepteurs (50) étant des moyens sélectivement activables d'un état de sommeil, où ils ne sont pas alimentés par une source d'énergie (34) du dispositif récepteur, vers un état opérationnel, où ils sont alimentés et aptes à filtrer, amplifier et démoduler les impulsions recueillies,
le dispositif récepteur comprenant, outre les moyens récepteurs, des moyens de réveil (66) aptes à discriminer la réception d'un signal spécifique de réveil configuré selon ledit motif caractéristique prédéterminé et, en réponse, faire basculer les moyens récepteurs de l'état de sommeil vers l'état opérationnel,
ensemble **caractérisé en ce que** :
- ladite communication sans fil est une communication par voie intracorporelle au moyen de signaux constitués desdites impulsions électriques, aptes à être conduites par les tissus interstitiels du corps ;
- ledit signal spécifique de réveil généré par lesdits moyens émetteurs est un train desdites impulsions, configuré selon un motif d'impulsions caractéristique prédéterminé (68, 70 ; 80, 82) ; et
- les moyens de réveil comprennent des moyens détecteurs (74, 84) et un comparateur à hystérésis (76 ; 88).

2. L'ensemble de la revendication 1, dans lequel les moyens de réveil sont dépourvus de circuit amplificateur.

3. L'ensemble de la revendication 1, dans lequel les impulsions du train d'impulsions spécifique de réveil (68, 70 ; 80, 82) sont des impulsions biphasiques comprenant chacune une alternance positive et une alternance négative.

4. L'ensemble de la revendication 1, dans lequel la consommation permanente des moyens de réveil est inférieure à 10 nW.

5. L'ensemble de la revendication 1, dans lequel les moyens émetteurs sont aptes à générer ledit motif caractéristique sous forme d'une impulsion initiale (68) d'amplitude élevée (A_{w}) pour le réveil, suivie d'une succession d'impulsions (70) de moindre amplitude (A) pour la communication avec le dispositif récepteur.

6. L'ensemble de la revendication 5, dans lequel les moyens émetteurs sont aptes à générer ledit motif caractéristique avec un délai de garde (T_{w}) séparant l'impulsion initiale d'amplitude élevée de la succession d'impulsions de moindre amplitude.

7. L'ensemble de la revendication 5, dans lequel les moyens détecteurs comprennent un détecteur de niveau de crête, ou crête-à-crête (74), des impulsions reçues.

8. L'ensemble de la revendication 5, dans lequel les moyens de réveil comprennent des moyens (78) aptes à inhiber le basculement des moyens récepteurs de l'état de sommeil vers l'état opérationnel sur détection d'une impulsion reçue de largeur (t) supérieure à une limite supérieure (T) prédéterminée.

9. L'ensemble de la revendication 1, dans lequel les moyens émetteurs sont aptes à générer ledit motif caractéristique sous forme d'une séquence spécifique d'impulsions (80, 82) d'amplitude uniforme (A) codant une valeur binaire prédéterminée formant une code de réveil, et les moyens de réveil comprennent des moyens (84-100) pour individualiser les impulsions reçues, décoder la valeur binaire codée par ces impulsions reçues, et vérifier si cette valeur décodée concorde ou non avec une valeur prédéterminée mémorisée dans le dispositif récepteur.

10. L'ensemble de la revendication 9, dans lequel les moyens émetteurs sont aptes à générer la séquence spécifique d'impulsions sous formes d'une pluralité de salves distinctes d'impulsions, certaines d'entre ces salves étant constituées d'un premier nombre d'impulsions (80) codant un '1' binaire et les autres salves étant constituées d'un second nombre d'impulsions (82), différent du premier nombre, codant un '0' binaire,
et dans lequel les moyens détecteurs comprennent un détecteur d'enveloppe (84) délivrant en sortie une série de valeurs binaires obtenues chacune par intégration d'une salve d'impulsions correspondante reçue, et des moyens numériques (96, 98, 100) de comparaison de cette série de bits à ladite valeur prédéterminée mémorisée.

11. L'ensemble de la revendication 10, dans lequel le détecteur d'enveloppe (84) comprend un circuit multiplicateur de tension (86).

12. L'ensemble de la revendication 10, dans lequel le second nombre d'impulsions (80) est double du premier nombre d'impulsions (82).

## Claims

1. Assembly comprising at least two active implantable medical devices, these devices (10, 12, 14, 16, 18) comprising means for communicating with one another wirelessly,
this assembly comprising at least one transmitter device and at least one receiver device, in which:
- the transmitter device comprises transmitter means (40) which can generate modulated pulse trains formed from a succession of pulses and apply these pulses to electrodes (22, 24) of the transmitter device,
said transmitter means (40) also being able, prior to the initiation of a communication with the receiver device, to generate a specific reactivation signal, configured according to a predetermined characteristic pattern; and
- the receiver device comprises receiver means (50) which can filter, amplify and demodulate pulse trains received at the terminals of electrodes (22', 24') of the receiver device,
said receiver means (50) being means which can be selectively activated from a sleep state, in which they are not powered by an energy source (34) of the receiver device, to an operational state, in which they are powered and can filter, amplify and demodulate the received pulses,
the receiver device comprising, in addition to the receiver means, reactivation means (66) which can discriminate the reception of a specific reactivation signal configured according to said predetermined characteristic pattern and, in response, switch over the receiver means from the sleep state to the operational state,
the assembly being **characterized in that**:
- said wireless communication is a communication by intracorporeal pathway by means of signals consisting of said electrical pulses, which can be conducted by the interstitial tissues of the body;
- said specific reactivation signal generated by said transmitter means is a train of said pulses, configured according to a predetermined characteristic pattern of pulses (68, 70; 80, 82); and
- the reactivation means comprise detector means (74, 84) and a hysteresis comparator (76; 88).

2. Assembly of Claim 1, in which the reactivation means have no amplifier circuit.

3. Assembly of Claim 1, in which the pulses of the specific reactivation pulse train (68, 70; 80, 82) are biphasic pulses each comprising a positive alternation and a negative alternation.

4. Assembly of Claim 1, in which the permanent consumption of the reactivation means is less than 10 nW.

5. Assembly of Claim 1, in which the transmitter means can generate said characteristic pattern in the form of an initial pulse (68) of high amplitude (A_{w}) for the reactivation, followed by a succession of pulses (70) of lesser amplitude (A) for the communication with the receiver device.

6. Assembly of Claim 5, in which the transmitter means can generate said characteristic pattern with a guard delay (T_{w}) separating the initial pulse of high amplitude from the succession of pulses of lesser amplitude.

7. Assembly of Claim 5, in which the detector means comprise a detector of peak level, or peak-to-peak level (74), of the pulses received.

8. Assembly of Claim 5, in which the reactivation means comprise means (78) which can inhibit the switchover of the receiver means from the sleep state to the operational state on detection of a received pulse of width (t) greater than a predetermined top limit (T).

9. Assembly of Claim 1, in which the transmitter means can generate said characteristic pattern in the form of a specific sequence of pulses (80, 82) of uniform amplitude (A) coding a predetermined binary value forming a reactivation code, and the reactivation means comprise means (84-100) for individualizing the received pulses, decoding the binary value coded by these received pulses, and checking whether this decoded value agrees or does not agree with a predetermined value stored in the receiver device.

10. Assembly of Claim 9, in which the transmitter means can generate the specific sequence of pulses in the form of a plurality of distinct bursts of pulses, some of these bursts consisting of a first number of pulses (80) coding a binary "1" and the other bursts consisting of a second number of pulses (82), different from the first number, coding a binary "0",
and in which the detector means comprise an envelope detector (84) delivering as output a series of binary values each obtained by integration of a corresponding received burst of pulses, and digital means (96, 98, 100) for comparing this series of bits to said stored predetermined value.

11. Assembly of Claim 10, in which the envelope detector (84) comprises a voltage multiplier circuit (86).

12. Assembly of Claim 10, in which the second number of pulses (80) is twice the first number of pulses (82).

## Patentansprüche

1. Einheit, die mindestens zwei aktive implantierbare medizinische Vorrichtungen enthält, wobei diese Vorrichtungen (10, 12, 14, 16, 18) Einrichtungen aufweisen, um drahtlos miteinander zu kommunizieren,
wobei diese Einheit mindestens eine Sendevorrichtung und mindestens eine Empfangsvorrichtung enthält, wobei:
- die Sendevorrichtung Sendeeinrichtungen (40) enthält, die Reihen modulierter Impulse erzeugen können, welche aus einer Folge von Impulsen bestehen, und diese Impulse an Elektroden (22, 24) der Sendevorrichtung anlegen können,
wobei die Sendeeinrichtungen (40) außerdem vor der Initiierung einer Kommunikation mit der Empfangsvorrichtung ein spezifisches Wecksignal erzeugen können, das gemäß einem vorbestimmten charakteristischen Muster konfiguriert ist; und
- die Empfangsvorrichtung Empfangseinrichtungen (50) enthält, die an den Elektrodenklemmen (22', 24') der Empfangsvorrichtung aufgenommene Impulsreihen filtern, verstärken und demodulieren können,
wobei die Empfangseinrichtungen (50) Einrichtungen sind, die selektiv aus einem Schlafzustand, in dem sie nicht von einer Energiequelle (34) der Empfangsvorrichtung gespeist werden, in einen betriebsfähigen Zustand aktivierbar sind, in dem sie gespeist werden und die aufgenommenen Impulse filtern, verstärken und demodulieren können,
wobei die Empfangsvorrichtung außer den Empfangseinrichtungen Weckeinrichtungen (66) enthält, die den Empfang eines spezifischen Wecksignals unterscheiden können, das gemäß dem vorbestimmten charakteristischen Muster konfiguriert ist, und als Antwort die Empfangseinrichtungen vom Schlafzustand in den betriebsbereiten Zustand umschalten können, wobei die Einheit **dadurch gekennzeichnet ist, dass**:
- die drahtlose Kommunikation eine Kommunikation auf intrakorporalem Weg mittels Signalen ist, die aus den elektrischen Impulsen bestehen, welche von den interstitiellen Geweben des Körpers geleitet werden können;
- das von den Sendeeinrichtungen erzeugte spezifische Wecksignal eine Reihe der Impulse ist, die gemäß einem vorbestimmten charakteristischen Muster von Impulsen (68, 70; 80, 82) konfiguriert ist; und
- die Weckeinrichtungen Erfassungseinrichtungen (74, 84) und einen Komparator mit Hysterese (76; 88) enthalten.

2. Einheit nach Anspruch 1, wobei die Weckeinrichtungen keine Verstärkungsschaltung aufweisen.

3. Einheit nach Anspruch 1, wobei die Impulse der spezifischen Reihe von Weckimpulsen (68, 70; 80, 82) Zweiphasen-Impulse sind, die je eine positive Halbperiode und eine negative Halbperiode enthalten.

4. Einheit nach Anspruch 1, wobei der durchgehende Verbrauch der Weckeinrichtungen geringer als 10 nW ist.

5. Einheit nach Anspruch 1, wobei die Sendeeinrichtungen das charakteristische Muster in Form eines Anfangsimpulses (68) hoher Amplitude (A_{w}) für das Wecken, gefolgt von einer Folge von Impulsen (70) geringerer Amplitude (A) für die Kommunikation mit der Empfangsvorrichtung erzeugen können.

6. Einheit nach Anspruch 5, wobei die Sendeeinrichtungen das charakteristische Muster mit einer Abstandszeit (T_{w}) erzeugen können, die den Anfangsimpuls hoher Amplitude von der Folge von Impulsen geringerer Amplitude trennt.

7. Einheit nach Anspruch 5, wobei die Detektoreinrichtungen einen Spitzenpegeldetektor oder Spitze-Spitze-Detektor (74) der empfangenen Impulse aufweisen.

8. Einheit nach Anspruch 5, wobei die Weckeinrichtungen Einrichtungen (78) enthalten, um das Umschalten der Empfangseinrichtungen vom Schlafzustand in den betriebsbereiten Zustand bei Erfassung eines empfangenen Impulses einer Breite (t) größer als eine vorbestimmte Obergrenze (T) zu verhindern.

9. Einheit nach Anspruch 1, wobei die Sendeeinrichtungen das charakteristische Muster in Form einer spezifischen Sequenz von Impulsen (80, 82) gleichmäßiger Amplitude (A) erzeugen können, die einen vorbestimmten Binärwert codieren, der einen Weckcode formt, und die Weckeinrichtungen Einrichtungen (84-100) enthalten, um die empfangenen Impulse zu vereinzeln, den durch diese empfangenen Impulse codierten Binärwert zu decodieren und zu überprüfen, ob dieser decodierte Wert mit einem in der Empfangsvorrichtung gespeicherten vorbestimmten Wert übereinstimmt oder nicht.

10. Einheit nach Anspruch 9, wobei die Sendeeinrichtungen die spezifische Sequenz von Impulsen in Form einer Vielzahl von unterschiedlichen Impulsgruppen erzeugen können, wobei bestimmte dieser Gruppen aus einer ersten Anzahl von Impulsen (80) bestehen, die eine binäre "1" codieren, und die anderen Gruppen aus einer zweiten Anzahl von Impulsen (82), anders als die erste Anzahl, bestehen, die eine binäre "0" codieren,
und wobei die Detektoreinrichtungen einen Hüllkurvendetektor (84), der am Ausgang eine Serie von Binärwerten liefert, von denen jeder durch Integration einer empfangenen entsprechenden Gruppe von Impulsen erhalten wird, und digitale Einrichtungen (96, 98, 100) zum Vergleich dieser Serie von Bits mit dem gespeicherten vorbestimmten Wert enthalten.

11. Einheit nach Anspruch 10, wobei der Hüllkurvendetektor (84) eine Spannungsmultiplikatorschaltung (86) enthält.

12. Einheit nach Anspruch 10, wobei die zweite Anzahl von Impulsen (80) das Doppelte der ersten Anzahl von Impulsen (82) ist.
